# EUROPEAN PATENT SPECIFICATION

(11) **EP 0 498 093 B1**
(45) Date of publication and mention of the grant of the patent: **03.01.1996**
(21) Application number: 91300857.9
(22) Date of filing: 01.02.1991
(51) Int. Cl.: A61B 17/06

(54) **Package for a combined surgical suture-needle device**
Verpackung für eine kombinierte chirurgische Nähfaden-Nadel-Vorrichtung
Empaquetage pour un dispositif chirurgical combinant un fil et une aiguille de suture

(43) Date of publication of application: 12.08.1992
(73) Proprietor: United States Surgical Corporation, Norwalk, Connecticut 06856 (US)
(72) Inventor: Brown, David L., Wallingford, Connecticut 06492 (US); Holzwarth, Henry A., Weston, Connecticut 06883 (US)
(74) Representative: Marsh, Roy David

(56) References cited:
- EP-A- 0 060 080
- EP-A- 0 471 441
- EP-A- 0 471 458
- EP-A- 0 494 081
- DE-A- 2 717 537
- DE-C- 3 246 162
- FR-A- 2 291 769
- US-A- 3 221 873
- US-A- 3 338 401
- US-A- 4 249 656
- US-A- 4 572 363

## Description

This invention relates to a package for a combined surgical suture-needle device. More particularly, this invention relates to a moisture-impervious sterile package featuring a peelable closure flap terminating in a tab which facilitates gripping of the flap in the course of its being opened.

Many types of packages for sutures and combined surgical suture-needle devices are known in the art including those described in U.S. Patent Nos. 2,917,878; 2,949,181; 2,965,225; 3,043,067; 3,143,209; 3,147,861; 3,163,288; 3,202,273; 3,221,873; 3,256,981; 3,280,971; 3,315,802; 3,319,782; 3,338,401; 3,357,549; 3,613,879; 3,627,120; 3,642,126; 3,648,949; 3,876,068; 3,939,969; 4,014,433; 4,069,912; 4,089,410; 4,135,623; 4,168,000; 4,249,656; 4,261,463; 4,284,194; 4,369,880; and, 4,549,649. Moisture-impervious packages possessing peelable, or strippable, closure flaps intended for the packaging of premoistened sheets are disclosed in U.S. Patent Nos. 4,131,195 and 4,192,420. A further package for a combined surgical suture-needle device is disclosed in DE-A-32 46 162, and forms the first part of claim 1

Most surgical suture-needle packages in current use consist of a folded paper surgical suture-needle retainer seated within a sterile outer envelope. The sterility of the surgical suture-needle and envelope are maintained by a second sealed overwrap. When the surgical suture-needle is to be used, the overwrap is opened in the operating room and the sealed envelope is placed within the sterile field. Sterile personnel then tear open the sterile envelope to gain access to the surgical suture-needle.

Efforts have been made to improve this type of package to facilitate removal of the surgical suture-needle therefrom. In one known type package, a portion of the inner suture retainer is secured to the sealed envelope so that the envelope and the inner retainer can be opened simultaneously and the end of the suture exposed for immediate pickup. A drawback to this arrangement is that after the envelope is torn open, the torn portion many times separates from the envelope and is loose in the operating room. In addition, the needle component is often difficult to locate.

In another known type of package, a peelable envelope features two laminates which are bonded to each other. An access area at the top of the envelope is provided to enable the user to open the envelope by peeling the laminates apart. A difficulty with this design is that the user must regrasp the envelope prior to removal of the suture, a time consuming and undesirable activity. Furthermore, there is no stop point when peeling open the envelope so that the envelope is frequently opened too far with the inner retainer falling out.

It is a principal object of the present invention to provide a package for a combined surgical suture-needle device which, in the sealed condition, reliably maintains its contents in a sterile condition for an indefinite period, is substantially moisture-impervious, accommodates a wide variety of sutures and surgical needle combinations, and is readily opened for convenient access of its contents which are consistently oriented in a way facilitating their removal from the package.

It is a particular object of the invention to provide such a package which accommodates a multitude of pocket-like, suture-needle retainer featuring an integral, peelable, i.e., strippable, closure flap terminating in a tab which facilitates the gripping of the flap in the course of its being peeled away to provide ready access to the contents of the package.

In keeping with these and other objects of the invention, there is provided a package for a combined surgical suture-needle device as defined in present claim 1.

The foregoing surgical suture-needle package possesses several advantages over known packages such as those described in the cited prior art. Thus, the peelable closure flap feature involves only one step to open the package, allowing access to the suture-needle combination contained therein without the need to regrasp the package, and results in less effort being expended to accomplish this than the tear feature of known types of surgical suture-needle packages. In the suture-needle device retainer member described herein to further illustrate the features of this present invention, a fold-over panel associated with the retainer and adhesively bonded to a portion of the interior surface of the peelable closure flap assures that following the opening of the package, the closure flap will remain secured to the remainder of the package. Such an arrangement represents a simple and effective solution to the over-tearing problem which can occur on opening the known types of surgical suture-needle packages referred to earlier.

In the accompanying drawings in which like numerals are used to refer to like elements throughout:
Fig. 1 is a top plan view of a package in accordance with this invention showing the closure flap in the closed condition;
Fig. 2 illustrates the package of Fig. 1 with the closure flap being peeled away to provide access to the package contents; and
Figs. 3 and 4 are obverse and reverse plan views, respectively, of a conventional combined suture-needle device retainer members which may be contained within the package of Fig. 1.

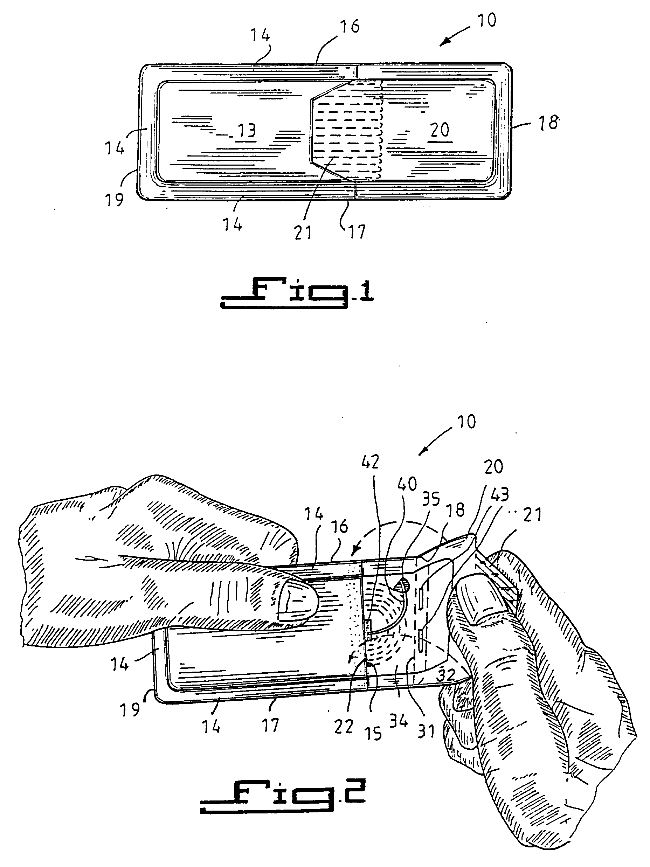

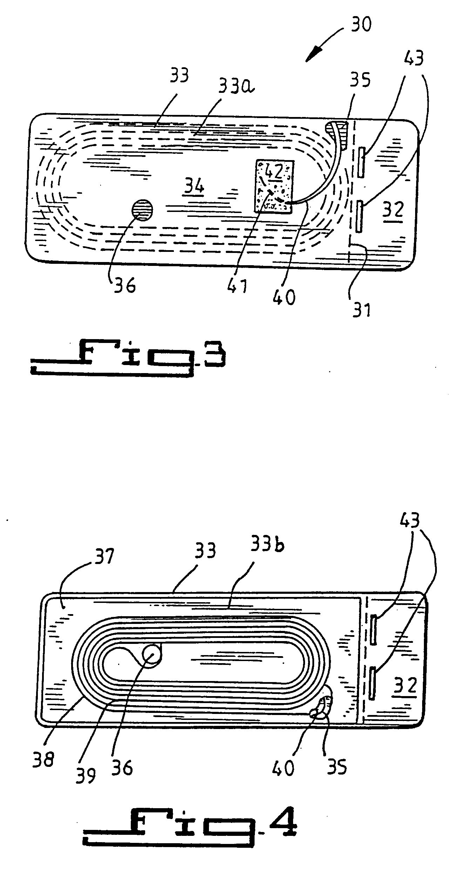

Referring to Figs. 1 and 2, suture package 10 comprises an outer envelope 11 made up of a first wall 12 and a second wall 13 adhered to each other along their common edges 14 employing any known and conventional adhesive.

Outer envelope 11 is characterized by longitudinal side edges 16 and 17 and top and bottom transverse edges 18 and 19.

The material of construction of walls 12 and 13 and peelable closure flap 20 is one which prevents or greatly impedes the transmission of moisture therethrough. In the embodiment shown, walls 12 and 13 and closure flap 20 are of laminate construction of a known type in which an aluminum foil is faced on its interior side with a polyolefin film such as polyethylene film. The laminate can vary in thickness from about 3 to about 5 mils and preferably from about 3.5 to about 4.5 mils.

The top transverse edge 22 of second wall 13 terminates a sufficient distance from perforate score line 31 of fold-over panel 32 of surgical suture-needle device retainer member 30 as to expose a portion of surgical needle 40 and foamed needle holder 42. Surgical suture-needle device retainer member 30 is a conventional retainer member. In the sealed condition of the package as shown in Fig. 1, peelable closure flap 20 completely seals pocket 15 of outer envelope 11 thus maintaining the contents of the package in the sterile condition. To facilitate the opening of sealed package 10, closure flap 20 is provided with a generally trapezoidal-shaped, knurled tab 21 which terminates some distance away from bottom transverse edge 19 of sealed envelope 11. Advantageously, the free edge of tab 21 terminates at a point which lies from about 20 to about 80%, and preferably from about 40 to about 60%, of the length of outer envelope 11 as measured from its top transverse edge 18. In the embodiment shown, this edge lies at a point which is about 50% of this length, i.e., at about the mid-point of the package. In addition to making it easier to grip closure flap 20, the knurling of tab 21 carries with it the advantage that any material shrinkage or deformation caused by the heat sealing of the flap will tend to go unnoticed.

To open sealed package 10, tab 21 is gripped in one hand, outer suture envelope 11 is gripped with the other and with a peeling or stripping motion, closure flap 20 is separated from its zone of adherent contact with walls 13 to expose needle 40. After package 10 has been opened, closure flap 20 remains hingedly attached to the top transverse edge 18 of outer envelope 11. To prevent an excessively forceful peeling motion from resulting in the complete detachment of closure panel 20 from transverse edge 18 of outer envelope 11, the reverse side of fold-over panel 32 is adhesively secured to a portion of the interior face of closure panel 20. In addition to providing this function, fold-over panel 32 in the closed condition of the package provides additional security against an accidental puncturing of the outer envelope by needle 40. Rectangular cutouts 43 in fold-over panel 32 facilitate the circulation of gaseous sterilizing agent, e.g., ethylene oxide, in and around needle 40 during the sterilization of the package contents.

Referring to Fig. 3, conventional surgical suture-needle device retainer member 30 includes a generally flat foil or sheet 33 having a main panel 34 and fold-over panel 32 joined to the main panel along perforate score line 31. The sharp tip 41 (indicated by the dotted line) of needle 40 is held within foamed needle holder 42 preventing or minimizing accidental puncture of outer envelope 11 (Fig. 1). An approximately triangular shaped aperture 35 is provided at one corner of main panel 34 through which the blunt tip of the needle and/or its attached suture passes. A circular aperture 36 is provided on main panel 34 in order to allow the suture to be vacuum-loaded into coiled passageway 38. To remove the combined surgical suture-needle device, grasping the exposed section of needle 40 by forceps, the tip of the needle is removed from foamed needle holder 42 and the needle with its attached length of suture is withdrawn from retainer member 30.

As shown in Fig. 4, a molded suture-retaining racetrack 37 possessing a coiled passageway 38 for suture 39 is mounted upon the reverse side of main panel 34. When fully positioned within pocket 15 of outer envelope 11 (Fig. 1), face 33b of suture-retaining reel 37 is in non-adherent contact with the inner surface of first wall 12 and the external surface 33a of main panel 33 is in non-adherent contact with the inner surface of second wall 13.

## Claims

1. A package (45) for a combined surgical suture-needle device which comprises:
a) an outer envelope (11) of substantially moisture impervious material comprising longitudinal side edges (16, 17) and top (18) and bottom (19) transverse edges, the envelope including first (12) and second (13) walls adhered to each other along their edges to define an accessible space (15) between them for receiving a combined surgical suture-needle device retainer member and a grip-facilitating tab (21) for separating the first and second walls to allow access to the space; and characterised in that:
b) the second wall (13) included a closure flap (20) hinged at a peripheral portion of said first (12) wall and sealing access to the space, the closure flap terminating in the grip-facilitating tab (21) for peeling back the closure flap;
c) the second wall (13) further includes a portion which terminates at a top transverse edge (22) spaced from the top edge (18) of the envelope (11) thereby to expose a portion of the space as a pocket (15);
d) the closure flap (20) is hinged at the top edge of the outer envelope so that, in use, the flap is peeled back to the top edge (18) to expose the pocket (15) portion.

2. A package as claimed in Claim 1 wherein the tab (21) is of heat shrink material and is knurled.

3. A package as claimed in Claim 1 or 2 wherein the outer envelope (a) and closure flap (b) are fabricated from a laminate.

4. A package as claimed in Claim 3 wherein the laminate includes an exterior surface layer of aluminium and an interior surface layer of a polyolefin.

5. A package as claimed in Claim 1, 2, 3 or 4 wherein the free edge of the grip-facilitating tab of closure flap (b) terminates at a point which lies at a distance of from about 20 to 80% of the length of the outer envelope as measured from the top transverse edge thereof.

6. A package as claimed in Claim 5 wherein the free edge of the grip-facilitating tab of closure flap (b) terminates at a point which lies at a distance of from about 40 to about 60% of the length of the outer envelope as measured from the top transverse edge thereof.

7. A package as claimed in any one of the preceding Claims which further comprises:
a retainer member received within the pocket of the package, the retainer member being adapted to retain at least one combined surgical suture-needle device.

8. A package as claimed in Claim 7 wherein combined surgical suture-needle device retainer member (c) includes means for exposing at least a portion of the needle and/or its attached suture upon opening of closure flap (b).

9. A package as claimed in Claim 8 wherein the retainer member possesses a fold-over panel protecting the needle, the fold-over panel being adhesively bonded to the closure flap such that upon opening of the closure flap, the flap remains secured to the fold-over panel.

10. A package as claimed in any one of the preceding claims, including at least one surgical suture-needle device arranged within the envelope such that the needle is in the said pocket portion which is exposed when the closure flap is peeled back.

## Patentansprüche

1. Packung (45) für eine kombinierte chirurgische Faden-Nadelvorrichtung mit:
a) einer äußeren Hülle (11) aus weitgehend feuchtigkeitsundurchlässigem Material, die Längsseitenränder (16, 17) und einen oberen (18) und einen unteren (19) Querrand aufweist, wobei die Hülle eine erste (12) und eine zweite (13) Wand, die entlang ihrer Ränder aneinanderklebt sind, um einen zugänglichen Raum (15) für die Aufnahme eines Halteelements für eine kombinierte chirurgische Faden-Nadelvorrichtung zwischen ihnen festzulegen, und eine das Greifen erleichternde Lasche (21) für die Trennung der ersten und zweiten Wand aufweist, um einen Zugang zu dem Raum zu gewähren; und
dadurch **gekennzeichnet,** daß:
b) die zweite Wand (13) eine Schließklappe (20) einschließt, die an einem Randabschnitt der ersten Wand (12) angelenkt ist und einen Zugang zu dem Raum verschließt, wobei die Schließklappe in der das Greifen erleichternden Lasche (21) zum Zurückziehen der Schließklappe endet;
c) die zweite Wand (13) ferner einen Abschnitt einschließt, der an einem oberen Querrand (22) endet, der von dem oberen Rand (18) der Hülle (11) beabstandet ist, um dadurch einen Abschnitt des Raumes als eine Tasche (15) freizulegen;
d) die Schließklappe (20) an dem oberen Rand der äußeren Hülle angelenkt ist, so daß im Gebrauch die Klappe zu dem oberen Rand (18) zurückgezogen wird, um den Taschen(15)-Abschnitt freizulegen.

2. Packung nach Anspruch 1, wobei die Lasche (21) aus einem Wärmeschrumpfmaterial besteht und gerändelt ist.

3. Packung nach Anspruch 1 oder 2, wobei die äußere Hülle (a) und die Schließklappe (b) aus einem Laminat hergestellt sind.

4. Packung nach Anspruch 3, wobei das Laminat eine äußere Flächenlage aus Aluminium und eine innere Flächenlage aus einem Polyolefin einschließt.

5. Packung nach Anspruch 1, 2, 3 oder 4, wobei der freie Rand der das Greifen erleichternden Lasche der Schließklappe (b) an einer Stelle endet, die in einem Abstand von etwa 20 bis 80% der Länge der äußeren Hülle, gemessen von ihrem oberen Querrand, liegt.

6. Packung nach Anspruch 5, wobei der freie Rand der das Greifen erleichternden Lasche der Schließklappe (b) an einer Stelle endet, die in einem Abstand von etwa 40 bis etwa 60% der Länge der äußeren Hülle, gemessen von ihrem oberen Querrand, liegt.

7. Packung nach einem der oben stehenden Ansprüche, ferner mit:
einem Halteelement, das innerhalb der Tasche der Packung aufgenommen ist, wobei das Halteelement wenigstens eine kombinierte chirurgische Faden-Nadelvorrichtung halten kann.

8. Packung nach Anspruch 7, wobei das Halteelement (c) für eine kombinierte chirurgische Faden-Nadelvorrichtung eine Einrichtung für das Freilegen wenigstens eines Abschnitts der Nadel und/oder ihres angebrachten Fadens nach dem Öffnen der schließklappe (b) einschließt.

9. Packung nach Anspruch 8, wobei das Halteelement eine Umklapptafel, die die Nadel schützt, besitzt, wobei die Umklapptafel haftend mit der Schließklappe verbunden ist, so daß nach dem Öffnen der Schließklappe die Klappe an der Umklapptafel befestigt bleibt.

10. Packung nach einem der oben stehenden Ansprüche, die wenigstens eine chirurgische Faden-Nadelvorrichtung aufweist, die innerhalb der Hülle angeordnet ist, so daß die Nadel in dem Taschenabschnitt ist, der freigelegt wird, wenn die Schließklappe zurückgezogen wird.

## Revendications

1. Empaquetage (45) pour un dispositif combiné chirurgical à fil de suture et à aiguille qui comprend :
a) une enveloppe extérieure (11) en un matériau sensiblement imperméable à l'humidité comprenant des bords latéraux longitudinaux (16, 17) et des bords transversaux supérieur (18) et inférieur (19), l'enveloppe incluant des première (12) et deuxième (13) parois qui adhèrent l'une à l'autre le long de leurs bords pour définir un espace accessible (15) entre celles-ci afin de recevoir un élément de retenue d'un dispositif combiné chirurgical à fil de suture et à aiguille et une patte facilitant la préhension (21) pour séparer les première et deuxième parois afin de permettre l'accès à l'espace; et caractérisé en ce que :
b) la deuxième paroi (13) inclut un volet de fermeture (20) articulé à une portion périphérique de ladite première paroi (12) et fermant hermétiquement l'accès à l'espace, le volet de fermeture se terminant par une patte facilitant la préhension (21) pour ouvrir par pelage le volet de fermeture;
c) la deuxième paroi (13) inclut en outre une portion qui se termine à un bord transversal supérieur (22) espacé du bord supérieur (18) de l'enveloppe (11) de façon à exposer une partie de l'espace comme une poche (15);
d) le volet de fermeture (20) est articulé au bord supérieur de l'enveloppe extérieure de telle façon qu'en cours d'utilisation le volet soit ouvert par pelage vers le bord supérieur (18) afin d'exposer la portion de poche (15).

2. Empaquetage selon la revendication 1, dans lequel la patte (21) est en un matériau thermo-rétractable et est moletée.

3. Empaquetage selon la revendication 1 ou 2, dans lequel l'enveloppe extérieure (a) et le volet de fermeture (b) sont fabriqués à partir d'un laminé.

4. Empaquetage selon la revendication 3, dans lequel le laminé inclut une couche de surface extérieure en aluminium et une couche de surface intérieure en polyoléfine.

5. Empaquetage selon la revendication 1, 2, 3 ou 4, dans lequel le bord libre de la patte facilitant la préhension du volet de fermeture (b) se termine à un point qui se situe à une distance d'environ 20 à 80 % de la longueur de l'enveloppe extérieure en mesurant depuis le bord transversal supérieur de celle-ci.

6. Empaquetage selon la revendication 5, dans lequel le bord libre de la patte facilitant la préhension du volet de fermeture (b) se termine à un point qui se situe à une distance d'environ 40 à environ 60 % de la longueur de l'enveloppe extérieure en mesurant depuis le bord transversal supérieur de celle-ci.

7. Empaquetage selon l'une des revendications précédentes, qui comprend en outre :
un élément de retenue reçu dans la poche de l'empaquetage, l'élément de retenue étant conçu pour retenir au moins un dispositif chirurgical combiné à fil de suture et à aiguille.

8. Empaquetage selon la revendication 7, dans lequel l'élément de retenue (c) du dispositif chirurgical combiné à fil de suture et à aiguille inclut un moyen pour exposer au moins une partie de l'aiguille et/ou de son fil de suture attaché lors de l'ouverture du volet de fermeture (b).

9. Empaquetage selon la revendication 8, dans lequel l'élément de retenue possède un panneau de repliage protégeant l'aiguille, le panneau de repliage étant lié par un adhésif au volet de fermeture de façon que lors de l'ouverture du volet de fermeture, le volet reste fixé au panneau de repliage.

10. Empaquetage selon l'une des revendications précédentes, incluant au moins un dispositif chirurgical à fil de suture et à aiguille disposé dans l'enveloppe de telle sorte que l'aiguille se trouve dans ladite portion de poche qui est exposée lorsque le volet de fermeture est ouvert par pelage.
